# EUROPEAN PATENT APPLICATION

(11) **EP 4 296 254 A1**
(43) Date of publication of application: **27.12.2023**
(21) Application number: 22756155.2
(22) Date of filing: 15.02.2022
(51) Int. Cl.: C07C 45/67, B01J 27/13, B01J 31/22, C07C 45/65, C07C 49/603, C07C 67/313, C07C 69/716, C07B 61/00

(54) **METHOD FOR PRODUCING CYCLOHEXENONE COMPOUND**

(30) Priority: 19.02.2021 JP 2021025335
(71) Applicant: API Corporation, Chikujo-gun, Fukuoka 871-0801 (JP)
(72) Inventor: NAGAHAMA, Masaki, Chikujo-gun, Fukuoka 871-0801 (JP); TANIIKE, Hirotsugu, Chikujo-gun, Fukuoka 871-0801 (JP); INOUE, Jun, Chikujo-gun, Fukuoka 871-0801 (JP)
(74) Representative: Godemeyer Blum Lenze Patentanwälte Partnerschaft mbB - werkpatent
(86) International application number: PCT/JP2022/005854
(87) International publication number: WO 2022/176837

(57) **Abstract**

A method for producing a cyclohexenone compound, comprising: an isomerization step of bringing a reaction raw material including the compound represented by the following formula (4) into contact with an isomerization catalyst in a solvent to obtain the compound represented by the following formula (3). Preferably, an addition step of reacting the compound (1) and methyl vinyl ketone in a solvent in the presence of a base to obtain compound (2); a cyclization step of reacting compound (2) in a solvent in the presence of a strong acid to obtain compound (3); and an isomerization step of bringing compound (4) contained as an impurity in the reaction product of the cyclization step into contact with an isomerization catalyst in a solvent are performed in order.

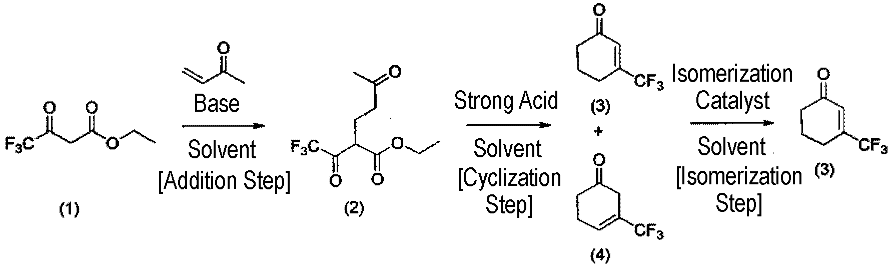

## Description

### Technical Field

The present invention relates to a method for producing a cyclohexenone compound.

### Background Art

Cyclohexenone compounds are useful as synthetic intermediates for various active pharmaceutical ingredients. For example, cyclohexenone compounds are useful as a synthetic intermediate for the nervous system drug disclosed in Patent Literature 1 or the like.

The method of Non Patent Literature 1 is known as a method for producing a cyclohexenone compound and its intermediate.

Non Patent Literature 1 discloses a method for producing 3-trifluoromethyl-2-cyclohexen-1-one by, as presented in the following reaction formula, reacting a starting material 2-cyclohexenone with (trimethylsilyl)trifluoromethane to produce 3-hydroxy-3-(trifluoromethyl)cyclohexan-1-one, which is further reacted with pyridinium chlorochromate (PCC).

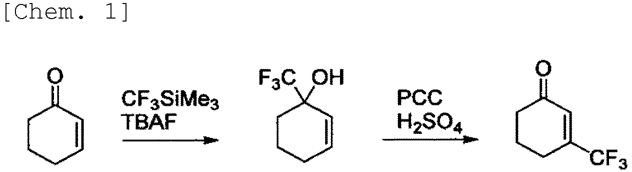

Unfortunately, the yield from this method is as insufficient as 28%, and a considerable amount of the isomer represented by formula (4) below is produced by a byproduct. Additionally, pyridinium chlorochromate, used here, is highly toxic and carcinogenic, and its waste is also hazardous, having a concern about increasing its environmental load.

Non Patent Literature 2 describes a method for producing an enone ester compound, as presented in the following reaction formula.

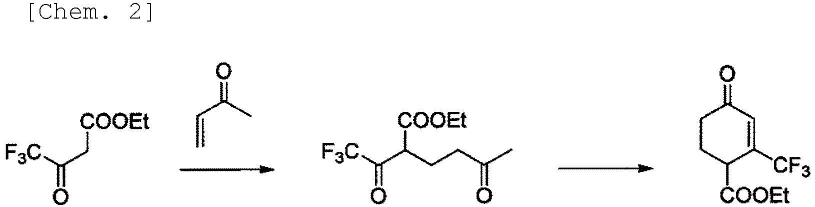

The enone ester compound thus obtained may be used in a method for producing cyclohexenone compounds in which the enone ester compound is de-esterified and decarboxylated according to the following reaction formula presented in Non Patent Literature 3.

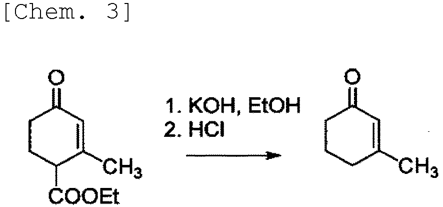

More specifically, in the method, 3-carboxy-1,1,1-trifluoroheptane-2,6-dione, and then further an enone ester compound, is produced from ethyl trifluoroacetoacetate and methyl vinyl ketone. The enone ester compound is de-esterified under basic conditions and then decarboxylated under acid conditions.

Unfortunately, when the method of Non Patent Literature 3 is applied for the compound targeted in the present invention, represented by formula (3) presented later, the reaction does not efficiently proceed, undesirably producing not only high-boiling-point impurities but also a large amount of isomeric byproduct.

In view of the above-described circumstances, the present inventors have investigated various methods for isomerizing the isomer represented by formula (4) by-produced in the synthesis of the compound represented by formula (3) into the targeted compound represented by formula (3). As a result, the inventors found that an isomerization reaction with a specific metal catalyst can reduce the amount of the isomer represented by formula (4) and efficiently recover the targeted compound represented by formula (3).

Non Patent Literature 4 described a method using rhodium (III) chloride as a catalyst of the following isomerization reaction of the aromatic compound presented below. However, the compound represented by formula (4), subject to the present invention, is not subjected to a favorable isomerization reaction even when using a rhodium-based catalyst, as will be described in Comparative Example 3 later.

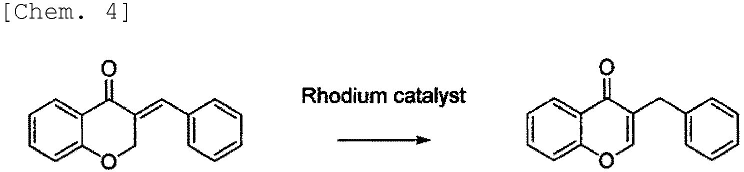

Patent Literature 1: International Publication No. WO 2018/038265
Non Patent Literature 1: J. Fluorine. Chem., 2000, 101, 199-202
Non Patent Literature 2: Synthetic Commun, 22 (4), 573-579 (1992)
Non Patent Literature 3: Angew. Chem. Int. Ed. 2014, 53, 2628-2632
Non Patent Literature 4: J. Chem. Soc., Perkin Trans. 1, 1977, 359-363

### Summary of Invention

The issue of the present invention is to provide a method for producing a cyclohexenone compound in high yield, at low cost, and with high productivity while reducing purification load and environmental load.

The present inventors found that a specific production method and purification method enable a cyclohexenone compound to be produced in high yield, at low cost, and with high productivity while reducing purification load and environmental load.

The gist of the present invention is as follows.

[1] A method for producing a cyclohexenone compound, comprising: an isomerization step of bringing a reaction raw material including the compound represented by the following formula (4) into contact with an isomerization catalyst in a solvent to obtain the compound represented by the following formula (3): 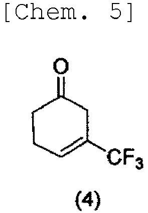 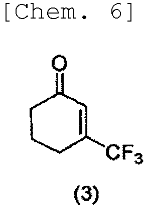
[2] The method according to [1] for producing a cyclohexenone compound, wherein the reaction raw material further includes the compound represented by the above formula (3).
[3] The method according to [2] for producing a cyclohexenone compound, comprising: a cyclization step of reacting the compound represented by the following formula (2) in a solvent in the presence of a strong acid to obtain the compound represented by the above formula (3) before the isomerization step: 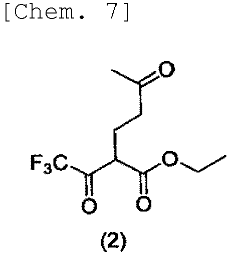
[4] The method according to [3] for producing a cyclohexenone compound, comprising: an addition step of reacting the compound represented by the following formula (1) with methyl vinyl ketone in a solvent in the presence of a base to obtain the compound represented by the above formula (2) before the cyclization step: 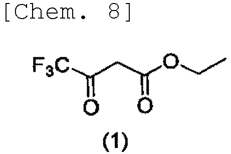
[5] The method according to any one of [1] to [4] for producing a cyclohexenone compound, wherein the isomerization catalyst is a ruthenium-containing catalyst.
[6] The method according to any one of [1] to [5] for producing a cyclohexenone compound, wherein the solvent is at least one selected from the group consisting of organic solvents and water.
[7] The method according to any one of [1] to [6] for producing a cyclohexenone compound, wherein the temperature at which the reaction raw material including the compound represented by the above formula (4) is brought into contact with the isomerization catalyst in the solvent is 10°C to 100°C.

### Effect of the Invention

According to the method for producing a cyclohexenone compound of the present invention, a cyclohexenone compound can be produced in high yield, at low cost, and with high productivity while reducing purification load and environmental load.

### Description of Embodiments

Hereinafter, embodiments of the method for producing a cyclohexenone compound of the present invention will be described in detail.

The method for producing a cyclohexenone compound of the present invention contains an isomerization step of bringing a reaction raw material including the compound represented by the following formula (4) into contact with an isomerization catalyst in a solvent to obtain the compound represented by the following formula (3).

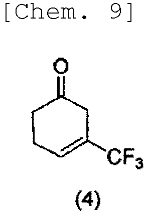

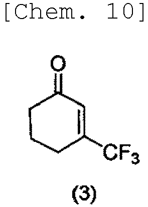

The method for producing a cyclohexenone compound of the present invention preferably contains a cyclization step of reacting the compound represented by the following formula (2) in a solvent in the presence of a strong acid to obtain the compound represented by the above formula (3) before the isomerization step. The method for producing a cyclohexenone compound of the present invention preferably contains an addition step of reacting the compound represented by the following formula (1) with methyl vinyl ketone in a solvent in the presence of a base to obtain the compound represented by the following formula (2) before the cyclization step.

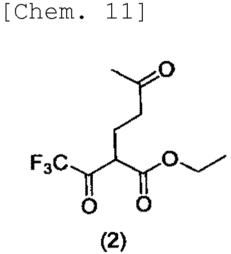

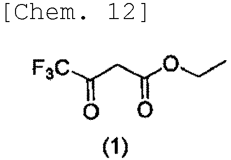

In the method of the present invention for producing a cyclohexenone compound, preferably, 3-trifluoromethyl-2-cyclohexen-1-one represented by formula (3) is produced by performing the following addition step, cyclization step, and isomerization step in order.

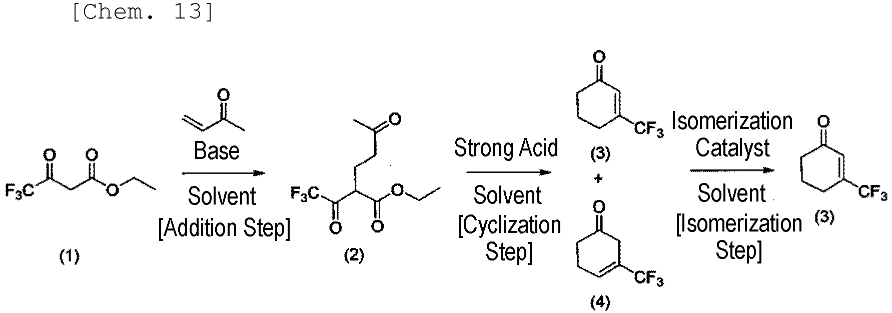

In the present invention, preferably, the following steps are performed in turn:
an addition step of reacting the compound represented by formula (1) (hereinafter also referred to as compound (1)) and methyl vinyl ketone in a solvent in the presence of a base to obtain the compound represented by formula (2) (hereinafter also referred to as compound (2));
a cyclization step of reacting compound (2) in a solvent in the presence of a strong acid to obtain the compound represented by formula (3) (hereinafter also referred to as compound (3)); and
an isomerization step of bringing the compound represented by formula (4) (hereinafter also referred to as compound (4)) contained as an impurity in the reaction product of the cyclization step into contact with an isomerization catalyst in a solvent.

The method of the present invention for producing a cyclohexenone compound will be described in the order of these steps.

### [Addition Step]

In the addition step, compound (1) and methyl vinyl ketone are reacted in a solvent in the presence of a base to obtain compound (2).

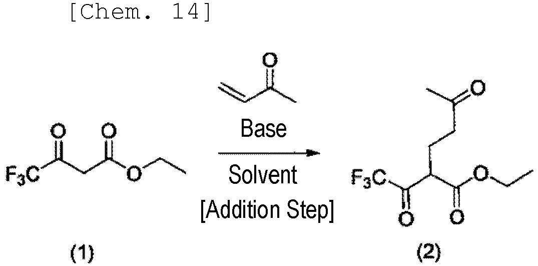

### <Reactive Substrate>

Compound (1), ethyl 4,4,4-trifluoroacetoacetate, may be a commercially available product or may be prepared in a known process.

The same applies to methyl vinyl ketone.

The amount of methyl vinyl ketone used can be equal to or greater than the reaction equivalent. The amount of methyl vinyl ketone used is typically 1 mol to 2 mol, preferably 1 mol to 1.5 mol, relative to 1 mol of compound (1) .

### <Base>

The base may be either an organic base or an inorganic base.

Examples of the organic base include triethylamine and other aliphatic amines; aniline and other aromatic amines; and heterocyclic amines, such as pyridine, lutidine, 1,8-diazabicyclo[5.4.0]-7-undecene, 1,5-diazabicyclo[4.3.0]-5-nonene, and 1,5,7-triazabicyclo[4.4.0]dec-5-ene.

Examples of the inorganic base include metal hydrides, metal hydroxides, and metal carbonates.

Metal hydrides include lithium hydride, sodium hydride, and potassium hydride.

Metal hydroxides include lithium hydroxide, sodium hydroxide, potassium hydroxide, and calcium hydroxide.

Metal carbonates include lithium carbonate, sodium carbonate, potassium carbonate, lithium bicarbonate, sodium bicarbonate, and potassium bicarbonate.

Such a base may be only one compound or a mixture of two or more compounds.

Among such bases, inorganic bases are preferable in view of reactivity, more preferably metal hydrides, particularly sodium hydride. Using a metal hydride leads to a reduced amount of base used.

For the amount of the base used relative to 1 mol of compound (1), the lower limit is typically 0.001 mol or more, preferably 0.005 mol or more, and more preferably 0.01 mol or more, and the upper limit is typically 2 mol or less, preferably 1 mol or less, and more preferably 0.5 mol or less.

Using the base in an amount equal to or more than the lower limit enables an efficient reaction. Using the base in an amount lower than or equal to the upper limit reduces the production of byproducts.

### <Solvent>

At least one selected from the group consisting of organic solvents and water is used as the solvent.

The organic solvent includes aliphatic hydrocarbon solvents such as hexane, cyclohexane, heptane and cycloheptane; aromatic hydrocarbon solvents such as toluene and xylene; aliphatic alcohol solvents having 1 to 8 carbon atoms such as methanol, ethanol, normal propanol, isopropanol, butanol, pentanol, hexanol, heptanol, octanol; ketone solvents such as methyl ethyl ketone and methyl isobutyl ketone; ether solvents such as diethyl ether, di-n-butyl ether, diisopropyl ether, di-n-butyl ether, methyl tert-butyl ether, cyclopentyl methyl ether , tetrahydrofuran, 2-methyltetrahydrofuran, dioxane, and the like.

From the viewpoint of reactivity, the solvent is preferably an organic solvent, more preferably an aromatic hydrocarbon solvent, and particularly preferably toluene.

For the amount of the solvent used relative to 1 kg of compound (1), the lower limit is typically 1L or more, preferably 2 L or more, and more preferably 3 L or more, and the upper limit is typically 20 L or less, preferably 15 L or less, and more preferably 10 L or less.

Using the solvent in an amount equal to or more than the lower limit enables an efficient reaction. Using the solvent in an amount lower than or equal to the upper limit enables the targeted compound to be produced with high productivity.

### <Reaction Conditions>

For the reaction temperature in the addition step, the lower limit is typically 0°C or more, preferably 10°C or more, more preferably 20°C or more, and particularly 30°C or more, and the upper limit is typically 80°C or less, preferably 70°C or less, more preferably 60°C or less, and particularly 50°C or less.

When the reaction temperature is equal to or more than the lower limit, the reactive substrate can react efficiently. When the reaction temperature is lower than or equal to the upper limit, the production of byproducts can be suppressed.

The reaction time is typically 0.1 hour to 24 hours and preferably 0.5 hour to 12 hours.

When the reaction time is equal to or more than the lower limit, the reaction can be efficiently conducted. When the reaction time is lower than or equal to the upper limit, the targeted compound can be produced with high productivity.

The reaction pressure is typically normal, but a certain degree of pressure may be applied.

### <Reaction Method>

When compound (1) and methyl vinyl ketone are reacted in a solvent in the presence of a base, these materials may be added in an appropriate order as desired. The materials may be added to the reaction system at once or in several batches.

For example, the base, the solvent, and compound (1), which is in liquid, are added into a reactor to prepare a uniform bed solution, into which methyl vinyl ketone in liquid is added under reaction conditions to prepare a uniform solution, where a reaction produces a liquid compound (2).

### <Work-up Process>

While the reaction liquid containing compound (2) may be applied to the next step as it is, the remaining base may be neutralized with an acid, followed by phase separation, and the thus obtained organic phase may be subjected to treatment such as filtration before being applied to the next step. The organic phase may be concentrated, and the resulting concentrate may be applied to the next step. Alternatively, the resulting product may be further purified by column chromatography or any other purification technique before being applied to the next step.

### [Cyclization Step]

In the cyclization step, compound (2) obtained in the addition step is reacted in a solvent in the presence of a strong acid to obtain compound (3).

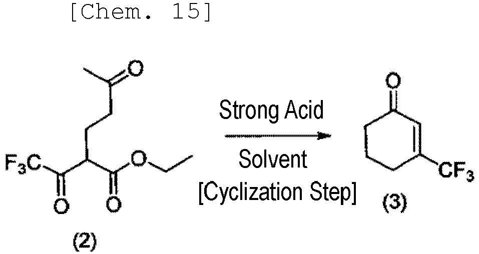

### <Reactive Substrate>

Compound (2), or 3-ethoxycarbonyl-1,1,1-trifluoroheptane-2,6-dione, may be obtained in the above-described addition step or may be produced by a known method.

### <Strong Acid>

The strong acid has an acid dissociation constant of less than 0 in water at 25°C. Examples of the strong acid that can be used include strong inorganic acids, such as sulfuric acid, hydrochloric acid, and nitric acid; and strong organic acids, such as methanesulfonic acid, trifluoromethanesulfonic acid, p-toluenesulfonic acid, and trifluoroacetic acid. Such a strong acid may be only one compound or a mixture of two or more compounds. Preferably, only one compound is used from the viewpoint of productivity.

Among those, inorganic acids, particularly sulfuric acid, are desirably used from the viewpoint of costs and reactivity. When sulfuric acid is used, the concentration of it is usually 30% by mass to less than 90%.

For the amount of the strong acid used relative to 1 mol of compound (2), the lower limit is typically 0.1 mol or more, preferably 0.2 mol or more, and more preferably 0.3 mol or more, and the upper limit is typically 20 mol or less, preferably 10 mol or less, and more preferably 5 mol or less.

Using the strong acid in an amount equal to or more than the lower limit enables an efficient reaction. Using the strong acid in an amount lower than or equal to the upper limit reduces the production of byproducts.

### <Solvent>

The solvent used in the cyclization step can be the same as those used in the above-described addition step. From the viewpoint of reactivity, organic solvents are preferred, more preferably alcohol solvents and ether solvents, particularly isopropanol and methyl tert-butyl ether. Such solvents may be used individually or in combination. It is, however, preferable to use only one solvent from the viewpoint of productivity.

For the amount of the solvent used relative to 1 kg of compound (2), the lower limit is typically 1 L or more, preferably 2 L or more, and more preferably 3 L or more, and the upper limit is typically 20 L or less, preferably 15 L or less, and more preferably 10 L or less.

Using the solvent in an amount equal to or more than the lower limit enables an efficient reaction. Using the solvent in an amount lower than or equal to the upper limit enables the targeted compound to be produced with high productivity.

### <Reaction Conditions>

For the reaction temperature in the cyclization step, the lower limit is typically 50°C or more, preferably 60°C or more, and more preferably 70°C or more, and the upper limit is typically 120°C or less, preferably 110°C or less, and more preferably 100°C or less.

When the reaction temperature is equal to or more than the lower limit, the reaction can be efficiently conducted. When the reaction temperature is lower than or equal to the upper limit, the production of byproducts can be suppressed.

The reaction time is typically 0.1 hour to 72 hours and preferably 12 hours to 36 hours.

When the reaction time is equal to or more than the lower limit, the reaction can be efficiently conducted. When the reaction time is lower than or equal to the upper limit, the targeted compound can be produced with high productivity.

The reaction pressure is typically normal, but a certain degree of pressure may be applied.

### <Reaction Method>

When compound (2) and the strong acid are reacted, these compounds may be added in an appropriate order as desired. The compounds may be added to the reaction system at once or in several batches.

For example, compound (2) and the solvent may be added together into a reactor to prepare a bed solution, into which the strong acid is added under reaction conditions for a reaction. Conversely, a solution of compound (2) may be added dropwise into a bed solution containing the strong acid.

### <Work-up Process>

In the cyclization step, compound (4) represented by the following formula (4), which is an isomer, is produced as a byproduct.

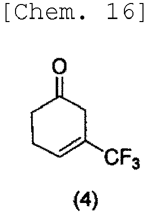

The reaction liquid containing compounds (3) and (4), in general, may be subjected to neutralization of the strong acid with a base and then treatment such as phase separation and filtration. Then, the organic phase containing compound (3) and compound (4) is distilled to remove the solvent, and the isolated compounds (3) and (4) are applied to the subsequent isomerization step. Alternatively, compounds (3) and compound (4) may be distilled from the organic phase containing compounds (3) and (4), and the distillates are applied to the subsequent isomerization step. These may be further purified by column chromatography or any other purification technique before the subsequent isomerization step.

### [Isomerization Step]

In the isomerization step, compound (4) is isomerized in a solvent in the presence of an isomerization catalyst.

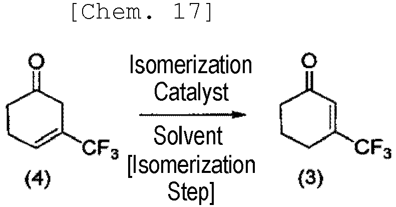

### <Reactive Substrate>

The starting material of the isomerization step, compound (4), may be high-purity 3-trifluoromethyl-3-cyclohexen-1-one. However, a mixture of compound (3) obtained in the above-described cyclization step and compound (4) is preferred in terms of an industrial process.

In the mixture containing compound (3) obtained in the cyclization step and compound (4), the proportion of compound (4) is typically 5 mol% to 50 mol% relative to compound (3). When this mixture is brought into contact with an isomerization catalyst, compound (4) is converted into compound (3) by an isomerization reaction, thus increasing the compound (3) content and reducing the compound (4) content. Thus, the amount of compound (4) in the reaction product liquid obtained after the isomerization reaction can be reduced to typically less than 5 mol%, preferably 4 mol% or less, particularly 3 mol% or less, relative to the amount of compound (3), resulting in a high yield of high-purity compound (3).

### <Isomerization Catalyst>

The isomerization catalyst can be any compound that can isomerize compound (4) to compound (3) without particular limitation. Preferred isomerization catalysts may be ruthenium-containing catalysts, particularly ruthenium-based catalysts or ruthenium-containing composite metal catalysts. Particularly, ruthenium-based catalysts are preferably used from the viewpoint of reactivity and costs. Such an isomerization catalyst may be only one compound or a mixture of two or more compounds. Preferably, only one compound is used from the viewpoint of productivity.

Ruthenium-based catalysts include ruthenium-supported carbon catalysts (hereinafter also referred to as "Ru/C"), ruthenium (III) chloride, tris(acetylacetonato)ruthenium (III), tris(2,2'-bipyridyl)ruthenium (II) chloride, and dichloro(p-cymene)ruthenium (II) (dimer).

Ruthenium-containing composite metal catalysts can be those containing ruthenium and enabling the reaction to proceed, and examples include platinum-ruthenium composite metal catalysts.

Among those catalysts, ruthenium (III) chloride and ruthenium-supporting carbon catalysts are preferred from the viewpoint of reactivity and costs. In the ruthenium-supporting carbon catalyst, the amount of ruthenium supported is preferably about 5% to 10% by mass relative to the carbon from the viewpoint of availability and costs.

For the amount of the isomerization catalyst used, in terms of metal in the catalyst, relative to 1 mol of compound (4), the lower limit is typically 0.001 mol or more, preferably 0.005 mol or more, and more preferably 0.01 mol or more, and the upper limit is typically 2 mol or less, preferably 1 mol or less, and more preferably 0.5 mol.

Using the isomerization catalyst in an amount equal to or more than the lower limit enables an efficient reaction. Using the isomerization catalyst in an amount lower than or equal to the upper limit reduces the production of byproducts.

### <Solvent>

The solvent used in the isomerization step can be the same as those used in the above-described addition step. From the viewpoint of reactivity and productivity, ether solvents, particularly methyl tert-butyl ether, are preferred. Such solvents allow an efficient isomerization reaction without inhibiting the reaction.

The solvent may be only one compound or a mixture of two or more compounds. Preferably, only one compound is used from the viewpoint of productivity.

For the amount of the solvent used relative to 1 kg of compound (4) or the mixture containing compound (3) and compound (4), the lower limit is typically 1 L or more, preferably 2 L or more, and more preferably 3 L or more, and the upper limit is typically 20 L or less, preferably 15 L or less, and more preferably 10 L or less.

Using the solvent in an amount equal to or more than the lower limit enables an efficient reaction. Using the solvent in an amount lower than or equal to the upper limit enables the targeted compound to be produced with high productivity.

### <Reaction Conditions>

For the reaction temperature in the isomerization step (the temperature at which the reaction raw material including the compound represented by formula (4) is brought into contact with the isomerization catalyst), the lower limit is typically 10°C or more, preferably 20°C or more, and more preferably 30°C or more, and the upper limit is typically 100°C or less, preferably 80°C or less, and more preferably 60°C or less.

When the reaction temperature is equal to or more than the lower limit, the reaction can be efficiently conducted. When the reaction temperature is lower than or equal to the upper limit, the production of byproducts can be suppressed.

The reaction time is typically 0.1 hour to 72 hours and preferably 1 hour to 24 hours.

When the reaction time is equal to or more than the lower limit, the reaction can be efficiently conducted. When the reaction time is lower than or equal to the upper limit, the targeted compound can be produced with high productivity.

The reaction pressure is typically normal, but a certain degree of pressure may be applied.

### <Reaction Method>

When compound (4) or the mixture containing compound (3) and compound (4) reacts with the isomerization catalyst, these compounds may be added in an appropriate order as desired. The compounds may be added to the reaction system at once or in several batches.

For example, the mixture containing compound (3) and compound (4) and the solvent are added together into a reactor to prepare a bed solution, and the isomerization catalyst is added under reaction conditions for a reaction.

### <Work-up Process>

The reaction liquid after the isomerization reaction is typically subjected to phase separation, filtration, or other treatment, and then, the targeted compound may be isolated from the organic phase by concentration, distillation, or any other isolation technique. After isolation, the targeted compound may be further purified by column chromatography or any other purification technique.

### EXAMPLES

The present invention will be further described in detail with reference to the following Examples. The invention is not limited to the Examples.

### [Abbreviation & Brevity Codes]

Abbreviation & Brevity codes used in the following Examples and Comparative Examples are as follows:
Compound (1): Ethyl 4,4,4-trifluoroactoacetate
Compound (2): 3-Ethoxycarbonyl-1,1,1-trifluoroheptane-2,6-dione
Compound (3): 3-Trifluoromethyl-2-cyclohexen-1-one
Compound (4): 3-Trifluoromethyl-3-cyclohexen-1-one
MTBE: Methyl tert-butyl ether

### [Analytical Method]

In the following Examples and Comparative Examples, the chemical purity (compound (3) and compound (4) contents (area%)) of the resulting compound was measured by gas chromatography (GC) under the following analytical conditions:

### <GC Analytical Conditions>

Analyzer: Agilent 6850, manufactured by Agilent
Column: DB-35, manufactured by Agilent
   (0.32 mm × 30m, 0.5 pm)
Injection volume: 1 µL
Injection way: Split (10:1)
Injection temperature: 200°C
Oven: 40°C (2 min) → (10°C/min) → 220°C (3 min)
Carrier gas: Helium, linear velocity: 33 cm/s
Detector: FID
   : H₂ 30 mL/min, Air 400 mL/min
Make-up gas: He 30 mL/min
Detector temperature: 250°C

### [Production Example 1]

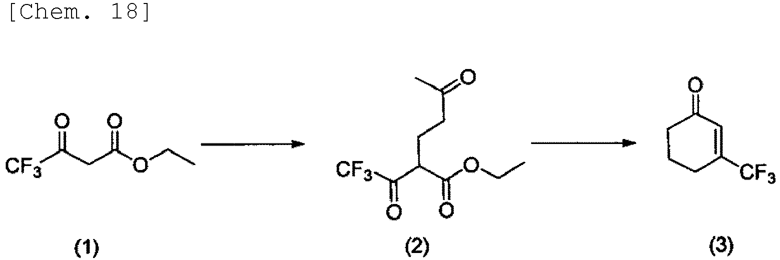

### [1] Production of Compound (2)

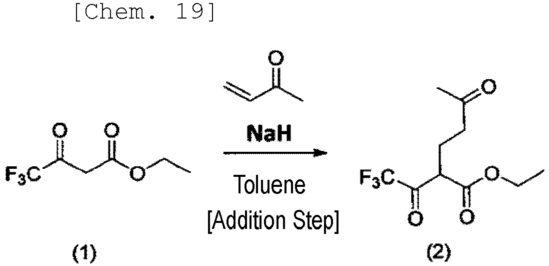

In a nitrogen atmosphere, 0.8 g (20.4 mmol) of 60 mass% sodium hydride and 650.2 g (750 mL) of toluene were stirred at 20°C in a separable flask. Then, 150 g (814.7 mmol) of compound (1) was added dropwise, followed by stirring, and the reaction liquid was heated to 40°C. After heating, 62.8 g (896.2 mmol) of methyl vinyl ketone was dropped, followed by a reaction for 5 hours.

After the reaction, the reaction liquid was cooled to 20°C, and 53.8 g (896.2 mmol) of acetic acid was added dropwise. Then, the reaction liquid was concentrated to a volume of about 300 mL under reduced pressure. After concentration, 592.5 g (750 mL) of isopropanol was added, and the reaction liquid was concentrated again to a volume of about 300 mL under reduced pressure. Thus, 238.4 g (90.3 area%, yield 94%) of a solution of compound (2) in isopropanol.

### [2] Production of Compound (3)

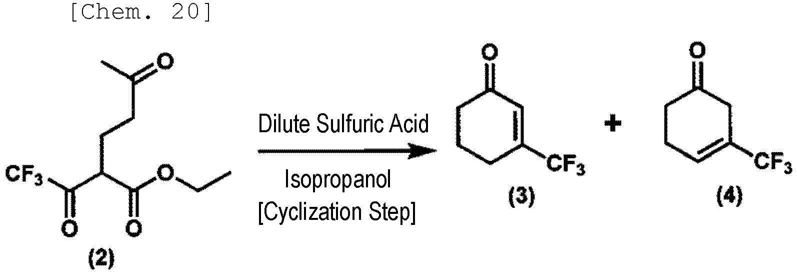

In a nitrogen atmosphere, 276 g of 98 mass% sulfuric acid and 300 g of water were stirred at 25°C in a separable flask. Then, 355.5 g of isopropanol and 238.4 g of the solution of compound (2) obtained in the above-described addition step were added dropwise, followed by stirring. Then, the reaction liquid was heated to 87°C, followed by a reaction for 22 hours. This reaction resulted in a solution of a mixture of compound (3) and compound (4) in isopropanol. In this process, the amount of sulfuric acid used is 3.4 mol relative to 1 mol of compound (2).

The solution of compound (3) and compound (4) in isopropanol, obtained in the above-described cyclization step was cooled to 25°C, and 900 g of water and 335.7 g of MTBE were added, followed by stirring. The reaction liquid was allowed to stand, thus obtaining an organic phase 1. Into the remaining water phase was added 335.7 g of MTBE. After stirring, the liquid was allowed to stand, and the water phase was removed to obtain an organic phase 2. The resulting organic phase 2 was mixed with organic phase 1, into which 450 g of water was added, followed by stirring. The mixture was allowed to stand, and the water phase was removed. Into the resulting organic phase was added 450 g of 5 mass% sodium bicarbonate solution. After stirring, the reaction liquid was allowed to stand, and the water phase was removed. Into the thus obtained organic phase was added 450 g of 1 mass% sodium chloride solution. After stirring, the reaction liquid was allowed to stand, and the water phase was removed. Into the thus obtained organic phase was added 78.3 g (814.9 mmol) of methanesulfonic acid dropwise. The reaction liquid was heated to 55°C, followed by a reaction for 3 hours.

After the reaction, the reaction liquid was cooled to 25°C and concentrated to a volume of about 450 ml at a reduced pressure of 100 hPa to yield a mixture (3-1) as the concentration residue (yield: 47%, compound (3): 61.8 area%, compound (4): 21.6 area %).

While being gradually heated from 25°C to 60°C, the resulting concentration residue, mixture (3-1), was concentrated at a reduced pressure of 30 hPa until no distillate was produced, thus obtaining a mixture (3-2) as distillate (yield: 39%, compound (3): 77.6 area%, compound (4): 15.2 area %).

The resulting distillate that is mixture (3-2) was subjected to batch rectification with 10 plates, a reflux ratio of 4, and a reduced pressure of 10 hPa while heated from 25°C to 100°C, thus obtaining a mixture (3-3) as an oily compound (compound (3): 83.5 area%, compound (4): 14.2 area%) .

The resulting mixture (3-3) was subjected to batch rectification with 10 plates, a reflux ratio of 4, and a reduced pressure of 10 hPa while heated from 25°C to 100°C, thus obtaining 22.39 g of compound (3) (yield throughout the process from compound (1): 18%, chemical purity: 98.8 area%, content: 98.2%, compound (4): 1.2 area%).

The analytical results of the thus obtained compound (3) are as follows:

### <Compound (3)>

GC-MS (CI: isobutene gas) : m/z = 164 (M⁺, 35), 136 (100), 67 (50)
¹H-NMR (CDCl₃, 400 MHz): δ = 2.13 (2H, m), 2.49 (4H, m), 6.37 (1H, s)

### [Example 1: Isomerization of compound (4) with isomerization catalyst: RuCl₃]

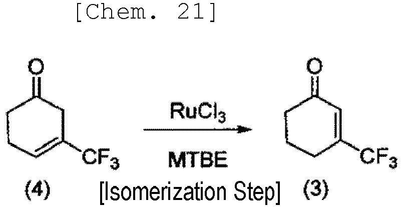

Into 350 mg of mixture (3-3) (mixture of compound (3) and compound (4), compound (4): 14.2 area%, compound (3): 83.5 area%) produced in Production Example 1, 2 ml of MTBE and 6 mg (0.01 mol equivalent to the substrate) of ruthenium (III) chloride were added. The solution was heated to 40°C, and a reaction proceeded for 26 hours. The resulting reaction liquid was subjected to GC analysis under the aforementioned analytical conditions, and the results were 92.4 area% for compound (3) and 2.0 area% for compound (4).

### [Example 2: Isomerization of compound (4) with isomerization catalyst: 5 mass% Ru/C]

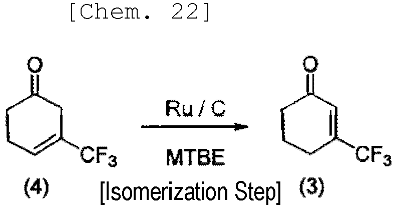

The isomerization was performed in the same manner as in Example 1, except that 6 mg of ruthenium (III) chloride (0.01 mol equivalent to the substrate) used in Example 1 was replaced with 86 mg of 5 mass% ruthenium/carbon (0.01 mol equivalent to the substrate). The resulting reaction liquid was subjected to GC analysis under the aforementioned analytical conditions, and the results were 94.0 area% for compound (3) and 2.3 area% for compound (4).

### [Example 3: Isomerization of compound (4) with isomerization catalyst: tris(acetylacetonato)ruthenium (III)]

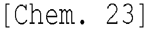

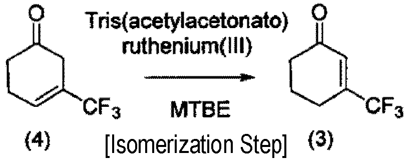

The isomerization was performed in the same manner as in Example 1, except that 6 mg of ruthenium (III) chloride (0.01 mol equivalent to the substrate) used in Example 1 was replaced with 9 mg of tris(acetylacetonato)ruthenium (III) (0.01 mol equivalent to the substrate). The resulting reaction liquid was analyzed under the aforementioned GC analytical conditions, and the results were 93.0 area% for compound (3) and 3.1 area% for compound (4).

### [Comparative Example 1: Isomerization of compound (4) with isomerization catalyst: palladium (II) chloride]

The isomerization was performed in the same manner as in Example 1, except that 6 mg of ruthenium (III) chloride (0.01 mol equivalent to the substrate) used in Example 1 was replaced with 1.6 mg of palladium (II) chloride (0.01 mol equivalent to the substrate). The resulting reaction liquid was analyzed under the aforementioned GC analytical conditions, and the results were 89.4 area% for compound (3) and 6.5 area% for compound (4). In this Comparative Example 1, the isomerization efficiency of compound (4) was low compared to that in Example 1 using ruthenium (III) chloride.

### [Comparative Example 2: Isomerization of compound (4) with isomerization catalyst: nickel (II) chloride]

The isomerization was performed in the same manner as in Example 1, except that 6 mg of ruthenium (III) chloride (0.01 mol equivalent to the substrate) used in Example 1 was replaced with 1.2mg of nickel (II) chloride (0.01 mol equivalent to the substrate). The resulting reaction liquid was analyzed under the aforementioned GC analytical conditions, and the results were 86.1 area% for compound (3) and 7.9 area% for compound (4).

### [Comparative Example 3: Isomerization of compound (4) with isomerization catalyst: rhodium (III) chloride]

Mixture (3-3), obtained in Example 1, was allowed to stand at room temperature for 2 weeks and subjected to analysis under the aforementioned GC analytical conditions. As a result, the resulting mixture (3-3A) exhibited 87.2 area% for compound (3), 9.4 area% for compound (4), and 3.4 area% for other impurities.

The isomerization was performed in the same manner as in Example 1 except that mixture (3-3) and 6 mg of ruthenium (III) chloride (0.01 mol equivalent to the substrate) used in Example 1 were replaced with a mixture (3-3A) and 9 mg of rhodium (III) chloride (0.01 mol equivalent to the substrate), respectively. The resulting reaction liquid was analyzed under the aforementioned GC analytical conditions, and the results were 87.6 area% for compound (3) and 8.6 area% for impurities (4).

The results of Examples 1 to 3 and Comparative Examples 1 to 3 are all presented together in the following Table 1.

**[Table 1]**

| | Isomerization catalyst | Compound (4) | | Compound (3) | | Compound (4)/compound (3) | | | Convers ion percent age [%] |
|---|---|---|---|---|---|---|---|---|---|
| | | Before reacti on [area% ] | After reacti on [area% ] | Before reacti on [area% ] | After reacti on [area% ] | Before reacti on [area% ] | After reacti on [area% ] | Differe nce | |
| Example 1 | Ruthenium (III) chloride | 14.2 | 2.0 | 83.5 | 92.4 | 17.0 | 2.2 | 14.9 | 85.9 |
| Example 2 | 5 mass% Ruthenium/carbon | 14.2 | 2.3 | 83.5 | 94.0 | 17.0 | 2.5 | 14.6 | 83.8 |
| Example 3 | Tris(acetylacetonato)ru thenium (III) | 14.2 | 3.1 | 83.5 | 93.0 | 17.0 | 3.3 | 13.7 | 78.2 |
| Comparative Example 1 | Palladium (II) chloride | 14.2 | 6.5 | 83.5 | 89.4 | 17.0 | 7.3 | 9.7 | 54.2 |
| Comparative Example 2 | Nickel (II) chloride | 14.2 | 7.9 | 83.5 | 86.1 | 17.0 | 9.2 | 7.8 | 44.4 |
| Comparative Example 3 | - | 14.2 | 9.4 | 83.5 | 87.2 | 17.0 | 10.8 | 6.2 | 33.8 |
| | Rhodium (III) chloride | 9.4 | 8.6 | 87.2 | 87.6 | 10.8 | 9.8 | 1.0 | 8.5 |

Examples 1 to 3 and Comparative Examples 1 to 3 in Table 1 suggest that the use of a ruthenium-containing catalyst enables compound (4) to isomerize efficiently to increase the compound (3) content and reduce the compound (4) content. In contrast, catalysts not containing ruthenium did not enable compound (4) to isomerize efficiently nor reduce the compound (4) content.

Although the present invention has been described in detail using specific embodiments, it will be apparent to those skilled in the art that various modifications can be made without departing from the spirit and scope of the present invention.

This application is based on Japanese Patent Application 2021-25335 filed on February 19, 2021, the entirety of which is incorporated by reference.

## Claims

1. A method for producing a cyclohexenone compound, comprising: an isomerization step of bringing a reaction raw material including the compound represented by the following formula (4) into contact with an isomerization catalyst in a solvent to obtain the compound represented by the following formula (3):

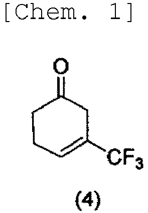

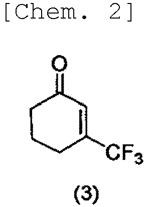

2. The method according to Claim 1 for producing a cyclohexenone compound, wherein the reaction raw material further includes the compound represented by the above formula (3).

3. The method according to Claim 2 for producing a cyclohexenone compound, comprising: a cyclization step of reacting the compound represented by the following formula (2) in a solvent in the presence of a strong acid to obtain the compound represented by the above formula (3) before the isomerization step:

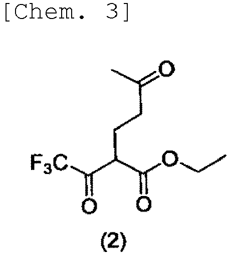

4. The method according to Claim 3 for producing a cyclohexenone compound, comprising: an addition step of reacting the compound represented by the following formula (1) with methyl vinyl ketone in a solvent in the presence of a base to obtain the compound represented by the above formula (2) before the cyclization step:

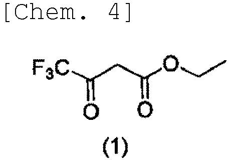

5. The method according to any one of Claims 1 to 4 for producing a cyclohexenone compound, wherein the isomerization catalyst is a ruthenium-containing catalyst.

6. The method according to any one of Claims 1 to 5 for producing a cyclohexenone compound, wherein the solvent is at least one selected from the group consisting of organic solvents and water.

7. The method according to any one of Claims 1 to 6 for producing a cyclohexenone compound, wherein the temperature at which the reaction raw material including the compound represented by the above formula (4) is brought into contact with the isomerization catalyst in the solvent is 10°C to 100°C.
